# EUROPÄISCHE PATENTANMELDUNG

(11) **EP 4 328 611 A1**
(43) Veröffentlichungstag der Anmeldung: **28.02.2024**
(21) Anmeldenummer: 22192157.0
(22) Anmeldetag: 25.08.2022
(51) Int. Cl.: G01R 33/565

(54) **VORRICHTUNG UND VERFAHREN ZUR STÖRUNTERDRÜCKUNG BEI EINEM MAGNETRESONANZTOMOGRAPHEN**

(71) Anmelder: Siemens Healthineers AG, 91301 Forchheim (DE)
(72) Erfinder: Düppenbecker, Peter Michael, 91074 Herzogenaurach (DE); Fackelmeier, Andreas, 91177 Thalmässing (DE); Tunea, Andrei-Vasile, 91054 Erlangen (DE)
(74) Vertreter: Siemens Healthineers Patent Attorneys

(57) **Zusammenfassung**

Die Erfindung betrifft eine elektronische Vorrichtung für einen Magnetresonanztomographen sowie ein System aus elektronischer Vorrichtung und Magnetresonanztomograph und ein Verfahren zum Betrieb. Die Vorrichtung weist einen Detektor zum Erfassen einer Störquelle auf. Mit dem erfassten Signal wird eine Bilderfassung entstört.

## Beschreibung

Die Erfindung betrifft eine elektronische Vorrichtung für einen Magnetresonanztomographen sowie einen Magnetresonanztomographen und ein Verfahren zum Betrieb. Die Vorrichtung weist einen Sensor zum Erfassen elektromagnetischer Störfelder auf. Mit dem erfassten Signal wird eine Bilderfassung entstört.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Empfangsantennen, sogenannte Lokalspulen verwendet, die zur Erzielung eines besseren Signal-Rauschabstandes unmittelbar am Untersuchungsobjekt angeordnet werden. Die Empfangsantennen können auch in einer Patientenliege verbaut sein.

Magnetresonanztomographen erfordern in zweierlei Hinsicht eine Hochfrequenzabschirmung. Zum einen werden zur Anregung der Kernspins Hochfrequenzimpulse mit Leistungen im Kilowattbereich erzeugt, die nur teilweise im Patienten absorbiert werden. Radiowellen, die die Patientendurchführung verlassen, werden in den Raum abgestrahlt und müssen daher zur Einhaltung von Emissionsgrenzwerten abgeschirmt werden.

Umgekehrt sind die für die Bildgebung zu empfangenden Magnetresonanzsignale extrem schwach. Um hier ein ausreichendes Signal-zu-Rausch-Verhältnis (SNR) zu erreichen, ist eine Abschirmung externer Störsignal erforderlich.

Deshalb werden im Stand der Technik um einen Magnetresonanztomographen aufwändige Schirmkabinen installiert, um sowohl Emissionen als auch Immissionen zu reduzieren.

Aus der Anmeldung WO 2019/068687 ist ein Verfahren und eine Vorrichtung zur Störunterdrückung in der Magnetresonanzabbildung bekannt.

Es ist daher eine Aufgabe der Erfindung, die Bildgebung in einer Umgebung mit störenden Hochfrequenzsignalen zu verbessern.

Diese Aufgabe wird von einer Vorrichtung nach Anspruch 1 sowie einem System nach Anspruch 5 und einem Verfahren nach Anspruch 6 gelöst.

Die erfindungsgemäße elektronische Vorrichtung ist für eine Verwendung gemeinsam oder als Teil eines Magnetresonanztomographen vorgesehen. Darunter ist insbesondere zu verstehen, dass die erfindungsgemäße elektronische Vorrichtung in Betrieb ist, während eine Bilderfassung mit dem Magnetresonanztomographen erfolgt. Die elektronische Vorrichtung kann beispielsweise eine Lokalspule sein, drahtlos oder über Kabel verbunden, oder auch ein medizinisches Gerät oder Sensor zur Überwachung des oder Kommunikation mit dem Patienten, ein Display, Terminal oder anderes Bedienelement zur Beobachtung oder Steuerung der Bilderfassung. Die elektronische Vorrichtung kann beispielsweise Komponenten aufweisen, die im Betrieb elektromagnetische Wechselfelder erzeugen und damit Störungen der Bilderfassung verursachen können. Dies können beispielsweise Prozessoren, digitale Schaltkreise, Oszillatoren oder auch Wechselrichter sein. Auch Schalter, elektronisch oder mechanisch, erzeugen impulsartige Störfelder.

Die Vorrichtung weist einen Detektor zum Erfassen einer Störquelle auf. Dieser Detektor kann wie nachfolgend zu den Unteransprüchen angeführt unmittelbar elektrische und/oder magnetische Störfelder der Störquelle detektieren. Es ist aber auch denkbar, dass die Störquelle indirekt erfasst wird. Beispielsweise kann die Aktivität eines Schaltkreises über die Stromversorgung überwacht werden und damit auf Störungen geschlossen werden, die durch diese Aktivitäten verursacht werden. Als Detektor könnte auch eine Logik oder eine Software dienen, die überwacht, welche Aktivitäten gerade in der Vorrichtung ausgeführt werden, entweder indem sie selbst diese Aktivitäten gestartet hat oder durch Zugriff auf eine Steuerung der elektronischen Vorrichtung diese verfolgt. Die Information weist dann eine Identifikation für die Aktivität auf.

Weiterhin weist die elektronische Vorrichtung einen Signalausgang auf. Der Signalausgang kann beispielsweise ein elektrisches Kabel und/oder Steckverbindung sein. Denkbar ist auch eine optische Kabelverbindung. Möglich wäre aber auch eine drahtlose Übertragung durch den freien Raum zwischen elektronischer Vorrichtung und dem Magnetresonanztomographen, beispielsweise mittels analoger oder digitaler Radioübertragung oder optische Übertragung im sichtbaren oder infraroten Wellenlängenbereich. Als Radioübertragung werden insbesondere auch Frequenzen oberhalb einer Larmorfrequenz des Magnetresonanztomographen angesehen, beispielsweise im GHz-Bereich, vorzugsweise in ISM-Bändern mit geringeren regulatorischen Einschränkungen. Denkbar wären auch elektrische Hochfrequenzübertragung durch induktive oder kapazitive Kopplung. Über den Signalausgang ist das Signal in Abhängigkeit von den elektromagnetischen Störfeldern zum Magnetresonanztomographen übertragbar.

Das erfindungsgemäße System weist eine erfindungsgemäße elektronischen Vorrichtung und einen Magnetresonanztomographen auf. Vorzugsweise ist die elektronische Vorrichtung Teil der Bilderfassung durch den Magnetresonanztomographen oder unterstützt diese beispielsweise durch zusätzliche Daten oder bei der Bedienung.

Der Magnetresonanztomograph des erfindungsgemäßen Systems weist einen Signaleingang auf. Der Signaleingang ist komplementär zu dem Signalausgang der elektronischen Vorrichtung ausgelegt, sodass der Magnetresonanztomograph über den Signalausgang der elektronischen Vorrichtung und den Signaleingang des Magnetresonanztomographen in Signalverbindung mit dem Sensor gebracht werden kann, um die Information über die Störquelle von dem Detektor der elektronischen Vorrichtung zu empfangen. Der Magnetresonanztomograph ist ausgelegt, eine Bilderfassung in Abhängigkeit von der Information des Detektors auszuführen. Beispielsweise kann die Information genutzt werden, um eine Erfassung bei Signalisieren einer Störung durch den Detektor zu unterbrechen und/oder zu wiederholen. Denkbar ist auch, entsprechend der Information einen Filter zu aktivieren. Ist die Information ein erfasstes Störsignal, so kann zum Reduzieren das erfasste Störsignal mit angepasster Amplitude und Phasenlage zur destruktiven Interferenz mit einem erfassten Magnetresonanzsignal gemischt werden.

Schließlich kann die Information gespeichert werden, um später bei der Bildrekonstruktion selbst Artefakte erkennen und/oder unterdrücken zu können. Beispielsweise kann mit einem zur Aktivität der Störquelle gespeichertem Referenzsignal und einer Information über den Zeitpunkt der Aktivität eine Korrelation zwischen Magnetresonanzsignal und dem gespeicherten Referenzsignal ermittelt werden und dieses dann aus den Magnetresonanzdaten entfern oder in der Bildrekonstruktion unterdrückt werden.

Auf vorteilhafte Weise ermöglicht das System aus der elektronischen Vorrichtung in Verbindung mit einem entsprechend zum Zusammenwirken mit der elektronischen Vorrichtung ausgelegten Magnetresonanztomographen eine besonders effektive und zuverlässige Unterdrückung von Störungen durch die elektrische Vorrichtung.

Das erfindungsgemäße Verfahren ist zum Betrieb eines erfindungsgemäßen Systems vorgesehen, um eine Magnetresonanzabbildung zu erzeugen.

In einem Schritt des Verfahrens wird ein hochfrequenter Anregungspulses von dem Magnetresonanztomographen über eine Antenne wie z.B. eine Körperspule oder Lokalspule in ein Untersuchungsobjekt bzw. einen Patienten ausgesendet, um darin ein hochfrequentes magnetisches Wechselfeld hervorzurufen, das Kernspins des Untersuchungsobjektes mit dem Magnetresonanztomographen in einem statischen bzw. quasi-statischen Magnetfeld einer Feldspule und/oder Gradientenspule zur Präzession anzuregen.

In einem weiteren Schritt des Verfahrens empfängt der Magnetresonanztomograph über eine Empfangsantenne, z.B. die Körperspule oder eine Lokalspule ein Magnetresonanzsignal des Untersuchungsobjektes für eine Bilderfassung.

In einem anderen Schritt empfängt der Magnetresonanztomograph über den Signaleingang eine Information zu der Störquelle. Dies kann eine Information zu einer Aktivität der Störquelle sein, die ein Störung verursacht oder direkt ein Signal zu einem elektromagnetischen Störfeld. Das Empfangen des Signals zu dem erfassten Störfeld kann dabei Empfangen eines analogen Signals des Sensors und dessen Weiterverarbeitung umfassen, aber auch eines beispielsweise in der elektronischen Vorrichtung vorbearbeiteten und/oder digitalisierten Signals sein. Das Empfangen kann auch das Speichern der Information in einem Speicher des Magnetresonanztomographen umfassen, insbesondere mit einem Zeitbezug zu dem empfangenen Magnetresonanzsignals.

In einem weiteren Schritt reduziert der Magnetresonanztomograph eine Auswirkung des elektromagnetischen Störfeldes bei der Bilderfassung in Abhängigkeit von dem Signal. Verschiedene Möglichkeiten sind bereits zuvor in Zusammenhang mit dem erfindungsgemäßen System beschrieben.

Das erfindungsgemäße Verfahren teilt die Vorteile des erfindungsgemäßen Systems.

Weitere vorteilhafte Ausführungsformen sind zu den Unteransprüchen angegeben.

In einer denkbaren Ausführungsform der erfindungsgemäßen elektronische Vorrichtung ist der Detektor ein Sensor zum Erfassen elektromagnetischer Störfelder, wie sie beispielsweise von den zuvor beschriebenen Komponenten der elektronischen Vorrichtung verursacht werden können. Der Sensor kann beispielsweise eine elektrische oder magnetische Antenne sein. Denkbar ist auch, dass der Sensor die elektromagnetischen Störfelder indirekt erfasst, beispielsweise indem er Spannungen oder Ströme in Leitern ohmsch, kapazitiv oder induktiv erfasst. Vorzugsweise erzeugt der Sensor ein Signal, dass abhängig von oder proportional zu einer Feldstärke der elektrischen und/oder magnetischen Komponente des Störfeldes oder dessen Hüllkurve ist.

Auf vorteilhafte Weise erlaubt die erfindungsgemäße elektronische Vorrichtung mittels des Sensors, elektromagnetische Störfelder unmittelbar an der Quelle in der elektromagnetischen Vorrichtung zu erfassen und so durch ein weitmöglich unverändertes Störsignal dessen spätere Unterdrückung zu erleichtern.

In einer denkbaren Ausführungsform der erfindungsgemäßen elektronische Vorrichtung ist der Sensor in unmittelbarer Nähe zu einer Quelle eines elektromagnetischen Störfeldes angeordnet. Vorzugsweise ist die Störquelle dabei selbst Teil der elektronischen Vorrichtung, beispielsweise Teil einer analogen oder insbesondere digitalen Aufbereitung und/oder Übertragungstechnik für das Magnetresonanzsignal. Insbesondere komplexe digitale Signalverarbeitungsbausteine weisen ein kaum vorhersagbares Spektrum an emittierten Frequenzen auf, dass sich auch mit den verarbeiteten Signalen auf nicht vorhersehbare Weise ändern kann. Ist der Sensor in unmittelbarer Nähe zu einer Störquelle angeordnet, nimmt er deren Signale aufgrund der Entfernungsabhängigkeit der elektrischen und/oder magnetischen Felder bevorzugt auf. Auch kann der Sensor beispielsweise mit Verbindungsleitungen der Quelle, d.h. der störenden elektronischen Komponente, elektrisch verbunden sein, um ein Störsignal auf diese Weise zu erfassen.

Auf vorteilhafte Weise ermöglicht ein Signal des Sensors, dass das Störsignal in besonders reiner Form darstellt, eine besonders effektive Entstörung der Bilderfassung.

In einer möglichen Ausführungsform der erfindungsgemäßen elektronischen Vorrichtung weist die elektronische Vorrichtung eine Abschirmung auf. Als Abschirmung wird dabei eine Anordnung angesehen, die elektrische und oder magnetische Felder, insbesondere hochfrequente Wechselfelder auf dem Weg in ein Inneres der elektronischen Vorrichtung bzw. wegen der Umkehrbarkeit auch bei der Ausbreitung von der Quelle in der elektronischen Vorrichtung in Ausbreitungsrichtung schwächt. Die Abschirmung weist vorzugsweise ein elektrisch leitendes Material auf. Das elektrisch leitende Material umschließt dabei die Quelle und den Sensor ganz oder vollständig. Beispielsweise kann die Abschirmung als Gehäuse der elektronischen Vorrichtung oder als separate Vorrichtung innerhalb oder außerhalb des Gehäuses den Sensor und die Quelle gemeinsam in einen von der Abschirmung umschlossenen Hohlraum einschließen. Denkbar ist auch, dass die Abschirmung eine oder mehrere Öffnungen für Durchführungen aufweist. Möglich ist auch eine Teilabschirmung, die Sensor und Quelle in nur eine oder mehrere von sechs möglichen Raumrichtungen umgibt. Zur Vermeidung von Wirbelströmen ist es auch denkbar, dass die Abschirmung Schlitze oder Löcher aufweist. Die Abschirmung kann aus einem elektrisch gut leitenden Material wie einem Metall ausgeführt sein. Vorzugsweise ist die Abschirmung dabei nicht ferromagnetisch. Vorzugsweise ist das Untersuchungsobjekt bzw. der Patient nicht innerhalb der Abschirmung, auch nicht mit einzelnen Körperteilen.

Denkbar ist aber auch ein leitender Kunststoff, wie beispielsweise kohlefaserhaltige Materialien. Die vergleichsweise geringere Leitfähigkeit reduziert Probleme durch Wirbelströme.

Indem der Sensor und die Quelle gemeinsam von einer Abschirmung zumindest teilweise umgeben sind, werden Magnetresonanzsignale des Untersuchungsobjekts auf dem Weg zum Sensor geschwächt, sodass der Sensor ein Signal liefert, dass nahezu frei bzw. unabhängig von Anteilen des Magnetresonanzsignals ist, wobei mit Magnetresonanzsignal an dieser Stelle nicht wie z.B. bei einer Lokalspule in der Vorrichtung von dem Magnetresonanzsignal abgeleiteten Signale gemeint sind, sondern die durch die Wechselfelder der Kernspins unmittelbar induzierten Ströme und Spannungen. Dieses fast reine Störsignal des Sensors reduziert auf vorteilhafte Weise Artefakte, die bei der nachfolgenden Störunterdrückung in der Signalverarbeitung oder Bildrekonstruktion durch Magnetresonanzsignalanteile verursacht werden, die fehlerhafter Weise als Störsignale interpretiert und verarbeitet werden.

In einer möglichen Ausführungsform des erfindungsgemäßen Verfahrens werden in einem Schritt Referenzdaten zu einer Störquelle erfasst. Diese Referenzdaten betreffen insbesondere die Emission von elektrischen und/oder magnetischen Störfeldern bei der einer vorbestimmten Aktivität der Störquelle. Im einfachsten Fall können die Referenzdaten Samples digitalisierter Störsignale bei der Aktivität sein. Möglich sind beispielsweise auch davon abgeleitete Daten wie gemittelte Werte oder spektrale Informationen.

Es werden dann in dem Schritt des Reduzierens einer Auswirkung der Störquelle die Auswirkungen der Aktivität in Abhängigkeit von den Referenzdaten reduziert.

Es ist beispielsweise denkbar, dass die Referenzdaten ein Sample einer Emission der Störquelle bei der vorbestimmten Aktivität sind. Vorzugsweise ist die Emission bei jeder Ausführung der Aktivität durch die Störquelle im Wesentlichen identisch. Dann kann z.B. mittels einer Autokorrelation, vorzugsweise unter Nutzung eines Zeitstempels aus der Information über die Aktivität, ein Anteil des durch die Aktivität verursachten Störsignals in den Magnetresonanzdaten erkannt und z.B. durch Subtraktion reduziert werden. Sind die Daten nicht immer identisch, kann dennoch z.B. unter Nutzung spezifischer spektraler Information eine Filterung und damit eine Reduktion erfolgen. Auch Verfahren des maschinellen Lernens und der künstlichen Intelligenz bzw. entsprechend trainierte neuronale Netzwerke können die Transformation eines gemessenen Störsignals und die resultierende Störung im Magnetresonanzsignal antizipieren und die Störung im erzeugten Bild reduzieren bzw. beheben.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines Magnetresonanztomographen mit einer erfindungsgemäßen elektronischen Vorrichtung;
- Fig. 2: eine schematische Darstellung unterschiedlicher erfindungsgemäßer elektronischer Vorrichtungen;
- Fig. 3: eine schematische Darstellung einer beispielhaften erfindungsgemäßen elektronischen Vorrichtung im Detail;
- Fig. 4: eine schematische Darstellung einer beispielhaften erfindungsgemäßen elektronischen Vorrichtung im Detail;
- Fig. 5: eine schematische Darstellung eines Ablaufplans einer Ausführungsform des erfindungsgemäßen Verfahrens.

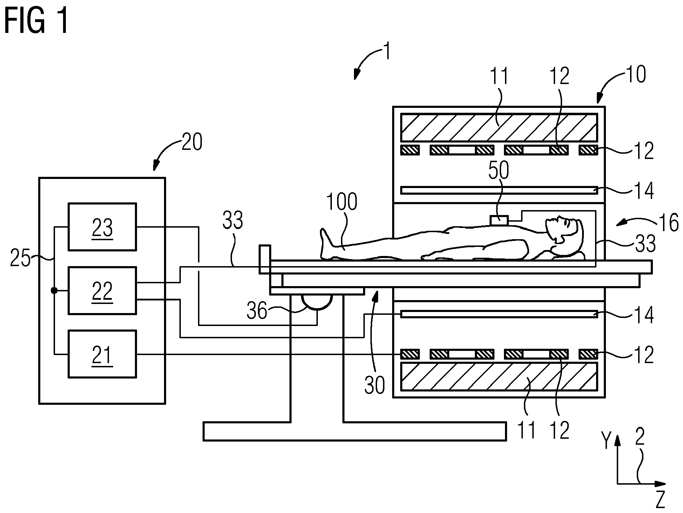

Fig. 1 zeigt eine schematische Darstellung einer Ausführungsform eines Magnetresonanztomographen 1 mit einer erfindungsgemäßen elektronischen Vorrichtung, hier einer Lokalspule 50.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Magnetfeldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 zeitlich und räumlich variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungssignale können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22.

Zum Empfang des Magnetresonanzsignals ist eine erfindungsgemäße Lokalspule 50 auf dem Patienten 100 im Patiententunnel 16 angeordnet, um Magnetresonanzsignale aus einem Untersuchungsbereich in unmittelbarer Nähe mit möglichst großem Rauschabstand zu erfassen. Die Lokalspule 50 ist über eine Anschlussleitung 33 mit einem Empfänger in der Hochfrequenzeinheit 22 in Signalverbindung.

Die Lokalspule 50 ist dabei eine elektronische Vorrichtung gemäß der Erfindung, die wie zu den nachfolgenden Figuren im Detail dargestellt, eine Störquelle und einen Sensor 70 aufweist. Der Sensor 70 ist dabei ausgelegt, von der Störquelle erzeugte elektromagnetische Störfelder zu erfassen und über die Anschlussleitung 33 als Signalverbindung eine Information zu den von der Störquelle erzeugten elektromagnetischen Störfeldern zu übermitteln, die geeignet ist, eine nachteilige Auswirkung auf die nachfolgende Signalverarbeitung und/oder Bildrekonstruktion zu reduzieren oder zu verhindern.

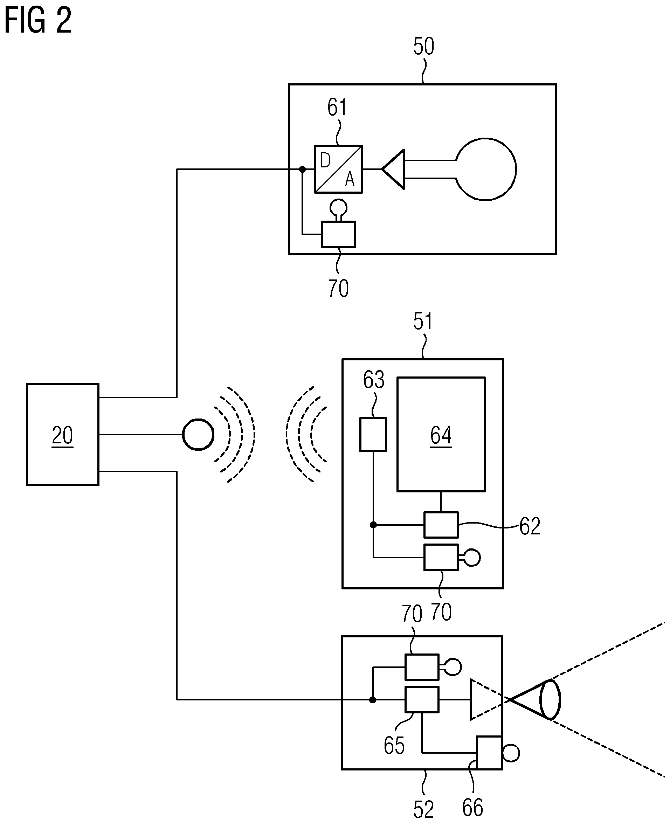

Fig. 2 zeigt eine schematische Darstellung unterschiedlicher erfindungsgemäßer elektronischer Vorrichtungen. Beispielhaft sind hier für erfindungsgemäße elektronische Vorrichtungen eine Lokalspule 50, ein Tablet-Computer 51 zur Bedienung und eine Überwachungskamera 52 zur Überwachung des und zur Kommunikation mit dem Patienten 100 während der Bilderfassung angegeben.

Die Lokalspule 50 kann als Störquelle beispielsweise einen Analog-Digitalwandler 61 aufweisen, der ein empfangenes Magnetresonanzsignal vor der Übertragung zum Magnetresonanztomographen 1 bzw. an die Hochfrequenzeinheit 22 der Steuereinheit 20 digitalisiert. Ein Sensor 70 ist in der Lokalspule 50 in der Nähe des A/D-Wandlers 61 angeordnet, um die von dem A/D-Wandler 61 erzeugten Störfelder möglichst unverfälscht und mit großem Störabstand zu erfassen. Der Sensor 70 kann beispielsweise die erfassten Störsignale in einer geschirmten Verbindungsleitung analog zu der Hochfrequenzeinheit 21 übertragen. Denkbar ist auch eine Digitalisierung und ein Multiplexen mit den digitalisierten Magnetresonanzdaten. Um Artefakte durch von dem Sensor 70 erfasste Magnetresonanzsignale zu verhindern, ist es beispielsweise auch denkbar, in Phasen ohne Magnetresonanzsignale die Signale des Sensors 70 zu erfassen, um Störsignale ohne Magnetresonanzsignalanteile zu erhalten und so diese von Magnetresonanzsignalen zu unterscheiden bzw. deren Charakteristiken zu erlernen.

Ein Tablet-Computer 51 ist hier beispielhaft als eine elektronische Vorrichtung mit drahtloser Anbindung über eine Funkeinheit 63 dargestellt. Die Funkeinheit 63 kann sich beispielsweise der digitalen Standards Bluetooth oder WLAN bedienen, denkbar sind aber auch andere zugelassenen Funkdienste, die die erforderliche Bandbreite bereitstellen, ausreichende Übertragungssicherheit bezüglich Datenverlustes bereitstellen und die gesetzlichen Regularien einhalten. Die meisten dieser Funkdienste beruhen auf digitaler Übertragung, wobei neben den zur Übertragung genutzten Wellenbereichen auch Störsignale durch die digitale Verarbeitung auf anderen störenden Frequenzbereichen wie z.B. auch der Larmorfrequenz des Magnetresonanztomographen 1 entstehen. In gleicher Weise erzeugen ein Prozessor 62 oder ein Display 64 Störfelder. Auch hier erfasst der Sensor 70 diese Störfelder unmittelbar am Entstehungsort und überträgt eine Information zur Störunterdrückung an die Steuereinheit 20, hier auf dem drahtlosen Weg über die Funkeinheit 63. Vergleichbares gilt für eine Lokalspule 50 mit drahtloser Übertragung.

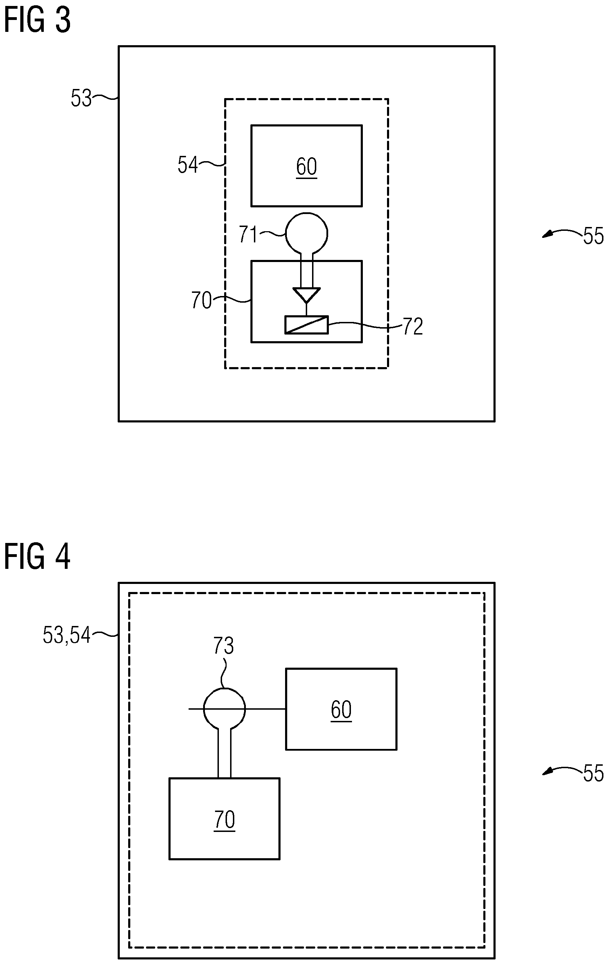

Fig. 3 zeigt eine schematische Darstellung einer beispielhaften erfindungsgemäßen elektronischen Vorrichtung im Detail. Die Darstellung ist hierbei generisch, die Störquellen 61, 62 63, 64, 65 der Fig. 2 sind hier durch die Störquelle 60 repräsentiert, die unterschiedlichen elektronischen Vorrichtungen 50, 51, 52 und weitere darüber hinausgehende durch die generische erfindungsgemäße elektronische Vorrichtung 55.

In Fig. 3 erfasst der Sensor 70 mittels einer Antennenschleife 71 durch Induktion ein störendes magnetisches hochfrequentes Wechselfeld, verstärkt dieses und digitalisiert es mittels eines A/D-Wandlers, um es zur Störunterdrückung an die Steuereinheit 20 des Magnetresonanztomographen zu übertragen. Denkbar ist es auch, mittels einer elektrischen Antenne wie einem Dipol oder Monopol ein störendes hochfrequentes elektrisches Wechselfeld zu erfassen. Über die Maxwellschen Feldgleichungen sind dabei die elektrische und die magnetische Komponente des hochfrequenten Wechselfeldes miteinander verknüpft, sodass die dargestellten Antennen auch jeweils eine Information für die andere Komponente bereitstellen.

In einer Ausführungsform der erfindungsgemäßen elektronischen Vorrichtung 55 weist diese eine Abschirmung 54 auf, die die Störquelle 60 und den Sensor 70 ganz oder teilweise umschließt. Auf diese Weise wird zum einen eine in den die elektrische Vorrichtung 55 umgebenden Raum abgestrahlte Hochfrequenzleistung der Störquelle 60 reduziert. Umgekehrt wird dadurch ein von dem Sensor 70 erfassten Magnetresonanzsignals reduziert bzw. minimiert. Wird in einer nachgeordneten Störunterdrückung oder Bildrekonstruktion dieser Magnetresonanzanteil als Störsignal behandelt, führt dies zu zusätzlichen Artefakten in den erfassten Bildern. Durch die Abschirmung 54 können so diese zusätzlichen Artefakte reduziert werden und/oder die Störunterdrückung durch die Trennung der Signale verbessert bzw. vereinfacht werden.

Indem die Abschirmung 54 wie in Fig. 3 nur die Störquelle 60 und den Sensor 70 umgibt, kann die Abschirmung 54 in den Abmessungen klein gehalten werden, sodass Auswirkungen auf die magnetischen Felder des Feldmagneten 11 und der Gradientenspulen 12 sowie die hochfrequenten elektromagnetischen Wechselfelder zur Anregung der Kernspins klein gehalten werden können. Darüber hinaus kann durch Wahl der geeigneten Materialien, bzw. Metalle, z.B. keine magnetischen bzw. ferromagnetischen Metalle oder Metalle mit geringer magnetischen Suszeptibilität oder der Geometrie, z.B. mit Schlitzen zur Vermeidung von Wirbelströmen, ein nachteiliger Effekt auf die Feldhomogenität geringgehalten werden. Dies ist besonders bei einer Lokalspule 50 als elektronische Vorrichtung wichtig, da diese während der Bilderfassung unmittelbar am zu erfassenden Bereich angeordnet wird.

In Fig. 4 ist eine weitere Ausführungsform einer erfindungsgemäßen elektronischen Vorrichtung dargestellt. Diese unterscheidet sich zum einen dadurch, dass hier Störfelder der Störquelle nicht durch ein in den Raum abgegebenes Wechselfeld erfasst wird, sondern das Störfeld durch die verursachenden Ströme oder Spannungen erfasst wird. Beispielhaft ist hier eine Induktionsschleife 73 um einen stromführenden Leiter dargestellt. Denkbar sind aber auch eine kapazitive Kopplung zum Erfassen von Spannungen oder die Verwendung von Richtkopplern zum Erfassen sich auf der Leitung ausbreitender hochfrequenter Signale, insbesondere auch deren Ausbreitungsrichtung. Letzteres kann auch zur Unterscheidung von Störungen genutzt werden, die von der Störquelle abgegeben werden oder in diese eingeleitet werden.

Weiterhin unterscheidet sich die Ausführungsform der Fig. 4 dadurch, dass die Abschirmung 54 nicht nur die Störquelle 60 und den Sensor 70 umgibt, sondern die ganze elektronische Vorrichtung 55. Es ist dabei denkbar, dass die Abschirmung 54 dabei auch gleichzeitig ein Gehäuse für die elektronische Vorrichtung bildet.

Es ist aber auch möglich, dass kein separater Sensor 70 als Detektor einer Störquelle bzw. deren erzeugter elektromagnetischer Störfelder vorhanden ist, sondern der Detektor Teil der elektronischen Vorrichtung ist. Beispielsweise kann die elektronische Vorrichtung eine Steuerung aufweisen, die selbst die Störquelle ist oder die Störquelle steuert. Die Steuerung kann dann als Detektor eine Information, z.B. über den Zeitpunkt und die Art der Aktivität an den Magnetresonanztomographen 1 bzw. dessen Steuerung 23 senden. Der Detektor kann auch als Programmelement auf der Steuerung der elektronischen Vorrichtung implementiert sein und beispielsweise Informationen über Art und Zeitpunkt von aktiven Unterprogrammen als Information senden.

Es sind darüber hinaus auch unterschiedliche Kombinationen der in Fig. 3 und Fig. 4 dargestellten Merkmale im Rahmen der erfindungsgemäßen elektronischen Vorrichtung denkbar.

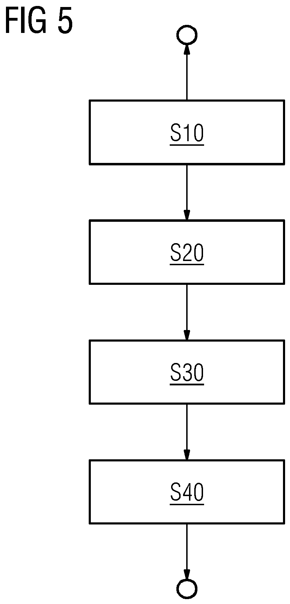

Fig. 5 zeigt eine schematische Darstellung eines Ablaufplans einer Ausführungsform des erfindungsgemäßen Verfahrens. Das Verfahren wird auf einem erfindungsgemäßen System mit einer erfindungsgemäßen elektronischen Vorrichtung ausgeführt, wie sie zu den vorhergehenden Figuren beschrieben wurden.

In einem Schritt S20 sendet die Hochfrequenzeinheit 21 unter Kontrolle der Steuerung 23 einen Anregungspuls für Kernspins eines Untersuchungsobjektes über die Körperspule 14 oder eine Lokalspule 50 aus.

In einem Schritt S30 empfangen die Hochfrequenzeinheit 21 über die Körperspule 14 oder vorzugsweise über die Lokalspule 50 als Empfangsantenne ein Magnetresonanzsignals des Untersuchungsobjektes durch den Magnetresonanztomographen 1. Üblicherweise weist dabei das empfangene Magnetresonanzsignal auch Störanteile auf, die von einer Störquelle 60 der elektronischen Vorrichtung 55 emittiert werden.

Um das Störsignal in dem empfangenen Magnetresonanzsignal zu reduzieren und/oder nachteilige Effekte durch das Störsignal bei einer nachfolgenden Bildrekonstruktion zu reduzieren, ist eine Kenntnis des oder Information über das Störsignal erforderlich.

In einem Schritt S40 empfängt der Magnetresonanztomograph 1 deshalb eine Information des Detektors 70 über die Störquelle 60.

Die Information kann beispielsweise ein Signal mit einer analogen oder digitalen Repräsentation der elektrischen und/oder magnetischen Feldstärke des Störsignal sein, wie es der Sensor 70 erfasst hat. Diese kann beispielsweise gewonnen werden, indem das elektromagnetische Störfeld mit einer Empfangsantenne von dem Sensor 70 empfangen wird. das Signal kann anschließend aufbereitet werden, beispielsweise verstärkt, gefiltert, in der Frequenz umgesetzt und/oder digitalisiert werden und von dem Sensor 70 zu der Hochfrequenzeinheit 21 übertragen werden, um dort in der weiteren Signalverarbeitung zur Reduktion von Störungen genutzt zu werden, wie es nachfolgend zum Schritt S50 beschrieben wird. Vorzugsweise wird dabei die zeitliche Relation bzw. Phasenbeziehung zu dem empfangenen Magnetresonanzsignal erhalten oder zusätzlich gespeichert.

Denkbar ist es auch, dass bereits eine Information über das Störsignal extrahiert wird. Beispielsweise kann der Zeitpunkt des Störsignals übertragen werden, um anschließend die gestörten Daten zu identifizieren und neu zu Erfassen oder zu interpolieren. Es ist auch denkbar, dass eine Frequenzinformation und oder Phaseninformation erfasst und übertragen wird, sodass bei der Bildrekonstruktion fehlerhafte Daten im k-Raum erkannt und korrigiert werden können.

In dem Schritt S50 wird anschließend die Auswirkung des Störsignals bzw. des elektromagnetischen Störfeldes der Störquelle bei der Bilderfassung mit Hilfe des Signals zu den erfassten elektromagnetischen Störfeldern reduziert.

Denkbar ist es beispielsweise, dass eine Phasenbeziehung und eine Amplitudenbeziehung zwischen einem Anteil des Störsignals in dem empfangenen Magnetresonanzsignal ermittelt wird. Dies kann z.B. durch Autokorrelation des empfangenen Magnetresonanzsignals und dem Signal des Sensors 70 ermittelt werden. Denkbar ist auch eine Kalibrierung, beispielsweise durch empfang eines "leeren" Magnetresonanzsignals, ohne zuvor anzuregen, sodass im Wesentlichen nur der Anteil des Störsignals übrigbleibt. Anhand der ermittelten Phasenbeziehung und Amplitudenverhältnisse kann so das Signal des Sensors 70 entsprechend gedämpft und verzögert werden, dass bei Addition des empfangenen Magnetresonanzsignals und des angepassten Sensorsignals eine destruktive Interferenz eintritt, die einen Anteil des Sensorsignals in dem Magnetresonanzsignals reduziert. Denkbar ist beispielsweise auch ein Optimierungsverfahren für die Verzögerung und Dämpfung, bei dem die Energie des Summensignals minimiert wird. Auch sind einfachere Verfahren denkbar, beispielsweise ein adaptiver Filter, dass die Frequenzen des Störsignals unterdrückt, was aber anschließend bei der Bildrekonstruktion berücksichtigt werden muss, um Artefakte zu vermeiden. Auch Verfahren der des maschinellen Lernens und der künstlichen Intelligenz können genutzt werden, um aus dem Signal des Sensors eine resultierende Störung im MR Signal vorherzusagen und z.B. durch destruktive Interferenz zu reduzieren.

Grundsätzlich ist es auch denkbar, dass in Schritt S50 erst bei der Bildrekonstruktion die Informationen zum Störsignal berücksichtigt werden. Beispielsweise kann über den Zeitpunkt und/oder Frequenz und Phasenlage des Störsignals die Lage gestörter Messpunkte im k-Raum identifiziert werden. Die Gestörten Messpunkte können dann beispielsweise interpoliert werden oder neu erfasst werden.

Ebenfalls möglich ist auch die Kombination beider Möglichkeiten, d.h. Unterdrückung im empfangenen Magnetresonanzsignal und zusätzliche Korrekturen bei der Bildrekonstruktion.

Insbesondere wenn eine Reduktion des Störsignals nicht in Echtzeit erfolgt, ist es denkbar, dass der Detektor nicht das Störsignal selbst erfasst, sondern lediglich einen Zeitpunkt und eine Information über die Störquelle sendet, beispielsweise über die von der Störquelle zu diesem Zeitpunkt ausgeführte Aktivität.

Vorzugsweise wurde dabei in einem Schritt S10 in einer Ausführungsform des Verfahrens Referenzdaten zu der Aktivität erfasst. Denkbar sind beispielsweise Samples der von der Störquelle bei der Aktivität erzeugten elektromagnetischen Störfelder, oder dafür charakteristische spektrale Informationen. Die Referenzdaten werden gespeichert. In Schritt S50 kann beispielsweise anhand der Information von dem Detektor ein Zeitfenster in den Magnetresonanzdaten identifiziert werden, das durch die Aktivität gestört sein kann. Mittels einer Autokorrelation von Referenzdaten und Magnetresonanzdaten kann in diesem Zeitfenster dann das Störsignal durch die Aktivität identifiziert und beispielsweise durch Subtraktion reduziert oder behoben werden. Sind die von der Aktivität hervorgerufenen Störungen nicht immer identisch, kann dennoch aufgrund charakteristischer Eigenschaften eine Reduktion erfolgen, beispielsweise durch Filterung von Frequenzbändern des Störsignals. Denkbar ist es auch, die in Schritt S10 erfassten Referenzdaten zum Training eines bereits erwähnten neuronalen Netzwerks zu nutzen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Elektronische Vorrichtung für einen Magnetresonanztomographen (1), wobei die elektronische Vorrichtung (55) einen Detektor zum Erfassen einer Störquelle (60) aufweist und einen Signalausgang zum Übertragen einer Information über die Störquelle (60) an den Magnetresonanztomographen (1).

2. Elektronische Vorrichtung für einen Magnetresonanztomographen (1), wobei der Detektor ein Sensor (70) zum Erfassen elektromagnetischer Störfelder ist und die Information die Störquelle eine Information über die erfassten Störfeldern ist.

3. Elektronische Vorrichtung nach Anspruch 2, wobei der Sensor (70) in unmittelbarer Nähe zu der Störquelle (60) eines elektromagnetischen Störfeldes angeordnet ist.

4. Elektronische Vorrichtung nach Anspruch 2 oder 3, wobei die elektronische Vorrichtung (55) eine Abschirmung (54) aufweist, wobei der Sensor (70) und die Störquelle (60) des elektromagnetischen Störfeldes von der Abschirmung (54) zumindest teilweise umgeben sind.

5. System aus einer elektronischen Vorrichtung (55) nach einem der vorhergehenden Ansprüche und einem Magnetresonanztomographen (1), wobei der Magnetresonanztomograph (1) einen Signaleingang aufweist, der ausgelegt ist, mit dem Signalausgang der elektronischen Vorrichtung (55) in Signalverbindung zu stehen, die Information über die Störquelle (60) von der elektronischen Vorrichtung (55) zu empfangen und eine Bilderfassung in Abhängigkeit von dem Signal auszuführen.

6. Verfahren zum Betrieb eines Systems nach Anspruch 5, wobei das Verfahren die Schritte aufweist:
(S20) Aussenden eines Anregungspulses für Kernspins eines Untersuchungsobjektes mit dem Magnetresonanztomographen (1);
(S30) Empfangen eines Magnetresonanzsignals des Untersuchungsobjektes durch den Magnetresonanztomographen (1) mit einer Empfangsantenne;
(S40) Empfangen einer Information zu der Störquelle (60) von dem Detektor durch den Magnetresonanztomographen (1);
(S50) Reduzieren einer Auswirkung der Störquelle (60) bei der Bilderfassung in Abhängigkeit von der Information zu der Störquelle (60).

7. Verfahren nach Anspruch 6, wobei in dem Schritt des Reduzierens eine Entstörsteuerung des Magnetresonanztomographen (1) einen von den elektromagnetischen Störfeldern der Störquelle (60) erzeugten Anteil in dem Magnetresonanzsignal reduziert.

8. Verfahren nach Anspruch 6, wobei in dem Schritt des Reduzierens der Magnetresonanztomograph (1) bei einer Bildrekonstruktion von der Störquelle (60) hervorgerufene Bildartefakte reduziert werden.

9. Verfahren nach einem der Ansprüche 6 bis 8, wobei in einem Schritt (S10) Referenzdaten zu einer Störquelle (60) erfasst werden und in dem Schritt (S50) Reduzieren einer Auswirkung der Störquelle (60) bei der Bilderfassung in Abhängigkeit den Referenzdaten erfolgt.

10. Computerprogrammprodukt, welches in eine Speichereinheit einer Steuerung (23) eines Magnetresonanztomographen (1) nach Anspruch 5 ladbar ist, aufweisend Programmcode-Mittel, um ein Verfahren nach einem der Ansprüche 6 bis 9 auszuführen, wenn das Computerprogrammprodukt in der Steuerung (23) des Magnetresonanztomographen (1) ausgeführt wird.

11. Computer-lesbares Medium, auf dem Programmcode-Mittel gespeichert sind, die von einer Steuerung (23) eines Magnetresonanztomographen (1) nach Anspruch 5 gelesen werden können und von der Steuerung (23) ausgeführt werden können, um ein Verfahren nach einem der Ansprüche 6 bis 9 auszuführen.
